# EUROPÄISCHE PATENTANMELDUNG

(11) **EP 3 701 979 A1**
(43) Veröffentlichungstag der Anmeldung: **02.09.2020**
(21) Anmeldenummer: 19159286.4
(22) Anmeldetag: 26.02.2019
(51) Int. Cl.: A61M 1/12, A61M 1/10

(54) **IMPLANTIERBARE BLUTPUMPE ZUM UNTERSTÜTZEN EINER HERZFUNKTION**

(71) Anmelder: Berlin Heart GmbH, 12247 Berlin (DE)
(72) Erfinder: PETERS, Oliver, 14129 Berlin (DE); WISNIEWSKI, Adrian, 10963 Berlin (DE); RICHERT, Dr.-Ing. Hendryk, 12487 Berlin (DE)
(74) Vertreter: Pfenning, Meinig & Partner mbB

(57) **Zusammenfassung**

Die vorliegende Anmeldung betrifft eine implantierbare Blutpumpe (2) zum Unterstützen einer Herzfunktion. Die Blutpumpe (2) umfasst eine Wärmequelle (24) und eine Wandung (9, 14), die einen Strömungskanal (10) begrenzt. Außerdem umfasst die Blutpumpe (2) einen Wärmeverteiler (13) zum Verteilen von durch die Wärmequelle (24) erzeugter Wärme auf eine Fläche der Wandung (9, 14). Der Wärmeverteiler (13) ist zur Übertragung von Wärme von der Wärmequelle (24) zu in dem Strömungskanal (10) gefördertem Blut thermisch leitfähig mit der Wärmequelle (24) und thermisch leitfähig mit einer von dem Strömungskanal (10) abgewandten Seite der Wandung (9, 14) verbunden.

## Beschreibung

Die vorliegende Anmeldung liegt auf dem Gebiet der Medizintechnik und insbesondere auf dem Gebiet implantierbarer Blutpumpen zur Unterstützung einer Herzfunktion. Die Anmeldung betrifft eine implantierbare Blutpumpe zum Unterstützen einer Herzfunktion.

Blutpumpen, insbesondere Herzpumpen, sind aus dem Stand der Technik bekannt. Diese Blutpumpen können zum Einsatz kommen, wenn eine Herzfunktion eines Patienten unterstützt oder ersetzt werden muss. Gängige Systeme, die hierbei verwendet werden, sind sogenannte VAD (ventricular assist devices). Derartige Blutpumpen können beispielsweise als sog. LVAD (left ventricular assist device), RVAD (right ventricular assist device) oder BiVAD (bi-ventricular assist device) ausgeführt sein. Diese Systeme umfassen neben der Blutpumpe, die im Betrieb in dem Patienten implantiert ist, in der Regel ein beispielsweise außerhalb eines Körpers des Patienten angeordnetes und mit der Blutpumpe über eine Leitung (Driveline) verbundenes Steuergerät. Die Blutpumpe umfasst in der Regel einen Motor mit einem Stator und einem mit einer Beschaufelung versehenen Rotor, der in einem Strömungskanal der Blutpumpe angeordnet ist. Durch von dem Steuergerät gelieferte Energie kann der Motor der Blutpumpe angetrieben werden, indem beispielsweise ein Stromfluss in Wicklungen des Stators erzeugt wird, durch den der Rotor mitsamt Beschaufelung zum Fördern von Blut des Patienten in Drehung versetzt wird. Beispielhaft wird auf den Stand der Technik der Druckschrift EP 3 181 163 A1 verwiesen.

Da vollimplantierbare VADs in der Regel durch einen ebenfalls implantierten Elektromotor angetrieben werden, spielt das Wärmemanagement eine wichtige Rolle bei der Entwicklung dieser Herzpumpen. Eine Verlustleistung des Motors sollte vollständig über den Blutstrom abgetragen werden. Blutführende Komponenten sollten sich dabei um nicht mehr als zwei Kelvin erwärmen. Um diese Vorgaben einzuhalten kann beispielsweise ein möglichst effizienter Motor eingesetzt und/oder der hydraulische Wirkungsgrad optimiert werden. Zur Verbesserung des Wirkungsgrads des Motors kann beispielsweise mehr Platz für die Spulen oder magnetisierbares Eisen reserviert werden, was jedoch zu einem größeren Platzbedarf des VADs führt.

Es ist eine Aufgabe der vorliegenden Anmeldung eine verbesserte implantierbare Blutpumpe zum Unterstützen einer Herzfunktion vorzuschlagen. Insbesondere soll die vorgeschlagene Blutpumpe eine Blutschädigung durch von der Blutpumpe erzeugter Wärme verringern. Zudem soll die vorgeschlagene Blutpumpe bei hohen Leistungen betreibbar und verkleinerbar sein, ohne dass eine Blutschädigung durch von der Blutpumpe erzeugte Wärme auftritt.

Diese Aufgaben werden gelöst durch eine implantierbare Blutpumpe mit den Merkmalen des Anspruchs 1. Vorteilhafte Weiterbildungen ergeben sich mit den Merkmalen der abhängigen Ansprüche und der Ausführungsbeispiele.

Die vorgeschlagene implantierbare Blutpumpe, insbesondere Herzpumpe, ist geeignet zum Unterstützen einer Herzfunktion. Die Blutpumpe umfasst eine Wärmequelle und eine Wandung, die einen Strömungskanal begrenzt. Außerdem umfasst die Blutpumpe einen Wärmeverteiler zum Verteilen von durch die Wärmequelle erzeugter Wärme auf eine Fläche der Wandung. Der Wärmeverteiler ist zur Übertragung von Wärme von der Wärmequelle zu in dem Strömungskanal gefördertem Blut thermisch leitfähig mit der Wärmequelle und thermisch leitfähig mit einer von dem Strömungskanal abgewandten Seite der Wandung verbunden.

Durch den Wärmeverteiler kann Wärme von der Wärmequelle auf die Wandung, die den Strömungskanal begrenzt, übertragen werden und an das durch den Strömungskanal geförderte Blut übertragen werden. Hierbei wird eine räumliche Verteilung der Wärme auf eine vergrößerte Fläche der Wandung erreicht und eine effektive Kühlfläche wird vergrößert. Somit wird das Blut auf weniger hohe Temperaturen erwärmt, sodass eine thermische Belastung bzw. Blutschädigung durch den Wärmeverteiler vermieden werden kann. Dadurch sind verkleinerte Blutpumpen möglich, die bei größerer Leistung und bei geringeren Volumenströmen betrieben werden können.

Typischerweise ist der Wärmeverteiler nicht vollständig in einem Bereich der Blutpumpe angeordnet, der direkt zwischen der Wärmequelle und dem Strömungskanal liegt. Der Wärmeverteiler kann zumindest teilweise in einem Bereich angeordnet sein, der in einer vom Strömungskanal abgewandten Richtung nicht von der Wärmequelle überdeckt wird. Auf diese Weise kann die effektive Kühlfläche besonders effizient vergrößert werden. Eine in radialer Richtung besonders kompakte Anordnung kann beispielsweise dadurch erreicht werden, dass der Wärmeverteiler vollständig in einem Bereich angeordnet ist, der in radialer Richtung, d.h. in der vom Strömungskanal abgewandten Richtung, nicht von der Wärmequelle überdeckt wird. Es kann beispielsweise vorgesehen sein, dass der Wärmeverteiler im Wesentlichen oder vollständig in einem in Bezug auf die Wärmequelle stromaufwärts- und/oder stromabwärtsliegenden Bereich angeordnet ist.

In typischen Ausführungen weist der Wärmeverteiler auf einer Seite, die der von dem Strömungskanal abgewandten Seite der Wandung zugewandt ist, eine Fläche von mindestens 50 mm², insbesondere zumindest 100 mm², auf. Über diese Fläche kann Wärme von dem Wärmeverteiler auf die Wandung übertragen werden. Auf diese Weise wird eine besonders effiziente Abfuhr der Wärme in das im Strömungskanal geförderte Blut erreicht, ohne dass eine Temperatur des Blutes lokal allzu stark erhöht wird. In einigen Ausführungen ist der Wärmeverteiler von im Wesentlichen flächiger Form ausgebildet. Eine Dicke des Wärmeverteilers kann beispielsweise zumindest 0,05 mm und/oder höchstens 3 mm, insbesondere höchstens 1 mm, betragen. Der Wärmeverteiler weist typischerweise auf der Seite, die der von dem Strömungskanal abgewandten Seite der Wandung zugewandt ist, eine Fläche von höchstens 100 cm² auf.

Es ist zudem in einigen Ausführungen vorgesehen, dass der Wärmeverteiler eine Wärmeleitfähigkeit aufweist, die größer ist als eine Wärmeleitfähigkeit der Wandung. Auf diese Weise wird erreicht, dass von der Wärmequelle auf den Wärmeverteiler übertragene Wärme auf dem Wärmeverteiler verteilt und großflächig über die Wandung an das Blut abgegeben wird. Eine dem Strömungskanal zugewandten Seite der Wandung weist typischerweise ein biokompatibles Material auf, insbesondere Titan oder eine Titanlegierung.

Es kann beispielsweise vorgesehen sein, dass der Wärmeverteiler ein Material mit einer Wärmeleitfähigkeit von zumindest 25 W/(m K), zumindest 50 W/(m K) oder zumindest 100 W/(m K), enthält. Beispielsweise kann der Wärmeverteiler ein Metall, insbesondere Aluminium oder Kupfer, enthalten. Um eine zuverlässige Verteilung der Wärme auf dem Wärmeverteiler zu gewährleisten, kann der Wärmeverteiler einstückig ausgebildet sein. In typischen Ausführungen ist der Wärmeverteiler nicht einstückig mit der Wandung und/oder mit der Wärmequelle ausgebildet. In einigen Ausführungen umfasst der Wärmeverteiler für eine effiziente Wärmeverteilung eine heat pipe bzw. ein Wärmerohr. Außerdem ist es in der Regel vorgesehen, dass der Wärmeverteiler nichtmagnetisch ist. Der Wärmeverteiler bildet typischerweise keinen Bestandteil des Stators und ist insbesondere keine Wicklung und kein Magnetkern des Stators.

In typischen Ausführungen weist die implantierbare Blutpumpe ein implantierbares Pumpengehäuse auf. Eine Außenwand des Gehäuses wird typischerweise durch ein biokompatibles Material, insbesondere Titan oder eine Titanlegierung, gebildet. Die Blutpumpe weist zudem in der Regel einen Motor auf. Der Motor umfasst typischerweise einen Stator, beispielsweise Wicklungen umfassend, und einen Rotor, beispielsweise einen Permanentmagneten umfassend. Der Motor ist typischerweise zumindest teilweise, insbesondere vollständig, in dem Pumpengehäuse aufgenommen. Zudem umschließt und/oder definiert das Pumpengehäuse den Strömungskanal. Der Rotor ist typischerweise in dem Strömungskanal angeordnet. Der Rotor ist in der Regel ebenfalls im Strömungskanal angeordnet. Das Pumpengehäuse weist in der Regel einen von dem Rotor stromaufwärts angeordneten Einlass und einen von dem Rotor stromabwärts angeordneten Auslass auf. Der Einlass kann eine Einlasskanüle umfassen. Typischerweise weist der Rotor eine Beschaufelung auf. Eine Drehung des Rotors ist in der Regel zum Fördern von Blut durch Erzeugen eines Stromflusses in den Wicklungen antreibbar. Es kann vorgesehen sein, dass die Wärmequelle in dem Pumpengehäuse aufgenommen ist. Zudem kann es vorgesehen sein, dass der Wärmeverteiler in dem Pumpengehäuse aufgenommen ist. Die Wandung kann eine Innenwandung des Pumpengehäuses sein.

Es kann in weiteren Ausführungen vorgesehen sein, dass die Wandung eine Außenwandung des Rotors ist. In diesem Fall ist der Wärmeverteiler typischerweise ebenfalls im Rotor aufgenommen. Auf diese Weise kann die Wärme über den Rotor auf das Blut im Strömungskanal übertragen werden.

Es kann vorgesehen sein, dass der Wärmeverteiler so angeordnet ist, dass dieser eingerichtet ist, die Wärme der Wärmequelle zu einem Bereich eines Hauptblutstroms im Strömungskanal zu übertragen. Der Bereich des Hauptblutstroms im Strömungskanal entspricht typischerweise dem Bereich des Strömungskanals, der beim Betrieb der Blutpumpe einen Strömungspfad mit dem größten Volumenstrom aufweist. In der Regel ist der Wärmeverteiler dort hinter der Wandung angeordnet und mit dieser in thermischem Kontakt stehend, wo der Hauptblutstrom im Strömungskanal an die Wandung angrenzt. Auf diese Weise kann die Wärme auf Bereiche übertragen werden, die durch das Vorliegen des Hauptblutstroms eine zuverlässige Wärmeabfuhr gewährleisten.

Es kann vorgesehen sein, dass die Wärmequelle ein Motorteil des Motors ist. Insbesondere kann es vorgesehen sein, dass die Wärmequelle ein Stator des Motors ist. Die Wärmequelle kann zudem eine Wicklung des Stators sein. Es kann aber auch vorgesehen sein, dass die Wärmequelle ein Spulenkern, insbesondere ein Eisenkern, des Stators ist. In anderen Ausführungen ist die Wärmequelle ein Lagerteil zur Lagerung des Rotors. Beispielsweise kann die Wärmequelle ein aktives Magnetlager zur Lagerung des Rotors oder ein Gleitlager zur Lagerung des Rotors sein. In anderen Ausführungen ist die Wärmequelle eine elektronische Steuereinheit zur Steuerung der Blutpumpe oder zum Auslesen von Sensoren.

Es kann in einigen Ausführungen auch vorgesehen sein, dass der Wärmeverteiler so angeordnet ist, dass dieser eingerichtet ist, die Wärme von kritischen Punkten im Strömungskanal fernzuhalten. Es kann beispielsweise vorgesehen sein, dass der Wärmeverteiler so angeordnet ist, dass dieser eingerichtet ist, die Wärme von zumindest einem Stagnationspunkt und/oder zumindest einem Rezirkulationspunkt fernzuhalten. Ein Stagnationspunkt entspricht einem Bereich des Strömungskanals, in dem ein Volumenstrom beim Betrieb in Bezug auf benachbarte Bereiche reduziert ist oder gänzlich zum erliegen kommt. Ein Rezirkulationspunkt entspricht einem Bereich des Strömungskanals, in dem dieselben Blutteilchen beim Betrieb mehrfach vorbeiströmen, beispielsweise infolge von Kreisströmungen. Sowohl Stagnationspunkte als auch Rezirkulationspunkte sind für einen Fachmann auch bei nicht betriebener Blutpumpe anhand der strukturellen Eigenschaften der Blutpumpe durch gängige Überlegungen zu erkennen. Auf beschriebene Weise wird somit erreicht, dass Blut an den Stagnations- und Rezirkulationspunkten nicht zu stark erwärmt wird.

Kritische Punkte, von denen die Wärme fernzuhalten ist, können in weiteren Ausführungen Verengungspunkte oder Punkte mechanischer Vorbelastung sein. Diese Bereiche können Bereiche des Strömungskanals sein, in denen das Blut, zum Beispiel durch Verengungen des Strömungskanals oder durch eine eine mechanische Kraftwirkung auf das Blut ausübende Beschaufelung eines Rotors, stärker mechanisch vorbelastet ist als in benachbarten Bereichen. Auch diese Bereiche des Strömungskanals sind durch einen Fachmann durch gängige Überlegungen anhand der strukturellen Eigenschaften einer nicht betriebenen Blutpumpe ohne Weiteres zu identifizierten.

Es kann des Weiteren vorgesehen sein, dass die implantierbare Blutpumpe einen thermischen Isolator aufweist. Der thermische Isolator kann in einem Bereich zumindest eines kritischen Punktes, insbesondere zumindest eines Stagnationspunktes und/oder zumindest eines Rezirkulationspunktes, zwischen der von dem Strömungskanal abgewandten Seite der Wandung und dem Wärmeverteiler angeordnet sein. Auf diese Weise kann durch den thermischen Isolator erreicht werden, dass im Bereich des kritischen Punktes eine reduzierte Wärmemenge von dem Wärmeverteiler auf das Blut übertragen wird. Eine Wärmeleitfähigkeit des thermischen Isolators beträgt typischerweise höchstens 1 W/(m K). Der thermische Isolator kann beispielsweise Kunststoff enthalten. Beispielsweise kann der thermische Isolator eine Folie, insbesondere eine Polyimid-Folie, sein. In weiteren Ausprägungen kann der thermische Isolator aus einer lokalen Verdickung der Wandung, beispielsweise einer Titanwandung, bestehen oder eine solche Verdickung umfassen.

Zudem kann es vorgesehen sein, dass die implantierbare Blutpumpe einen zweiten thermischen Isolator aufweist, der zwischen der Wärmequelle und einer Außenwand des Pumpengehäuses angeordnet ist. Auf diese Weise wird erreicht, dass die Wärme in den Strömungskanal und nicht in das Pumpengehäuse umgebendes Gewebe abgegeben wird. Hierdurch wird vermieden, dass eine Schädigung des das Pumpengehäuse umgebenden Gewebes auftritt.

Ein Abstand zwischen dem Wärmeverteiler und der Wärmequelle beträgt in typischen Ausführungen höchstens 5 mm, insbesondere höchstens 2 mm, sodass eine hinreichende Wärmeübertragung zwischen der Wärmequelle und dem Wärmeverteiler gewährleistet ist. Insbesondere kann es vorgesehen sein, dass ein Abstand zwischen zumindest einer Wicklung des Stators und dem Wärmeverteiler höchstens 5 mm, insbesondere höchstens 2 mm, beträgt. Auch kann es zur zuverlässigen Wärmeübertragung vorgesehen sein, dass ein Abstand zwischen der Wandung und dem Wärmeverteiler höchstens 5 mm, insbesondere höchstens 2 mm, beträgt. Außerdem kann eine Wärmebrücke vorgesehen sein. Der Wärmeverteiler kann über die Wärmebrücke mit der Wärmequelle verbunden sein. Die Wärmebrücke ermöglicht einen verbesserten Wärmeübergang zwischen der Wärmequelle und dem Wärmeverteiler und somit eine verbesserte Wärmeverteilung. Der Wärmeverteiler ist in typischen Ausführungen von der Wärmequelle, insbesondere von der zumindest einen Wicklung, elektrisch isoliert. Es kann beispielsweise vorgesehen sein, dass die Wärmebrücke elektrisch isolierend ist. Beispielsweise kann die Wärmebrücke eine Wärmeleitpaste, einen Wärmeleitkleber, ein Metall oder eine Keramik umfassen. Es kann auch vorgesehen sein, dass die Wandung über eine Wärmeleitpaste, einen Wärmeleitkleber oder eine Keramik mit dem Wärmeverteiler verbunden ist. Es kann auch vorgesehen sein, dass ein elektrischer Isolator, insbesondere eine Kunststofffolie, zwischen der Wärmebrücke und der Wandung und/oder zwischen der Wärmequelle und der Wandung vorgesehen ist, damit eine elektrische Isolierung der Wandung sichergestellt ist.

Es kann vorgesehen sein, dass der Wärmeverteiler die Innenwandung des Pumpengehäuses über einen Winkelbereich von zumindest 30 Grad, insbesondere zumindest 90 Grad, 180 Grad oder 270 Grad umgibt. Es kann auch vorgesehen sein, dass der Wärmeverteiler die Innenwandung vollständig umgibt. Auf diese Weise können eine große Fläche für den Wärmeübergang und ein effizienter Wärmeübertrag an die Wandung und das Blut im Strömungskanal erreicht werten. Ein Querschnitt des Strömungskanals kann beispielsweise rund sein. In derartigen Ausführungen ist der Wärmeverteiler typischerweise ringsegmentförmig oder ringförmig.

Das Pumpengehäuse kann zudem eine Volute umfassen. Ein Bereich der Volute kann den Auslass des Pumpengehäuses bilden. Ein durch die Volute begrenzter Teil des Strömungskanals kann im Wesentlichen spiralförmig sein. Zudem kann das Pumpengehäuse einen weiteren zylinderförmigen Teil des Strömungskanals aufweisen, der sich an den durch die Volute begrenzten Teil anschließt. Es kann in einigen Ausführungen vorgesehen sein, dass die Innenwandung eine Innenwandung der Volute ist. Somit kann durch den Wärmeverteiler Wärme von der Wärmequelle zu der Innenwandung der Volute übertragen werden. Vorteilhaft ist hierbei, dass im Bereich der Volute eine große Fläche für einen Wärmeübertrag zur Verfügung steht und dass das Blut im Bereich der Volute vergleichsweise große Volumenströme aufweist. Somit kann auf diese Weise ein besonders effizienter Wärmeübertrag bei geringer Blutschädigung erreicht werden.

Ausführungsbeispiele werden nachfolgend anhand der Abbildungen beschrieben. Es zeigen
- Fig. 1: eine schematische Ansicht einer Blutpumpe, die in einem Körper eines Patienten implantiert ist,
- Fig. 2: eine weitere schematische Ansicht der Blutpumpe,
- Fig. 3: eine schematische Detailansicht der Blutpumpe,
- Fig. 4: eine weitere schematische Ansicht der Blutpumpe,
- Fig. 5: eine schematische Ansicht eines Wärmeverteilers,
- Fig. 6: eine Illustration eines Wärmeflusses in der Blutpumpe sowie
- Fig. 7: eine Ansicht einer Blutpumpe gemäß einem weiteren Ausführungsbeispiel.

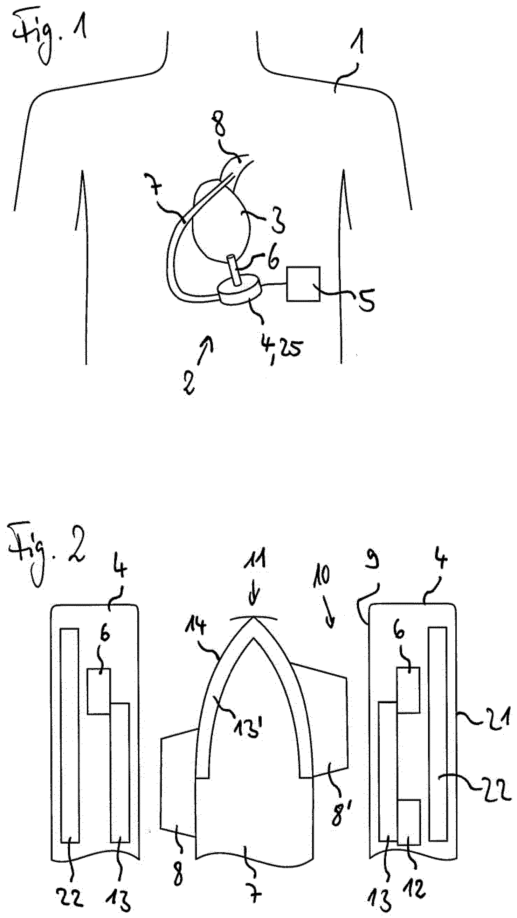

Figur 1 zeigt schematisch einen Körper 1 eines Patienten, in dem eine Blutpumpe 2 zur Unterstützung einer Funktion des Herzens 3 implantiert ist. Die Blutpumpe 2 weist einen typischerweise als Elektromotor mit einem Rotor ausgeführten Motor auf, der in einem biokompatiblen Pumpengehäuse 4 der Blutpumpe 2 aufgenommen ist. Ein Teil des Pumpengehäuses 4 ist verbreitert und als Volute 25 ausgebildet. Das Pumpengehäuse 4 ist mit einem Steuergerät 5 verbunden, das ebenfalls implantiert sein kann, wie schematisch gezeigt ist. Das Steuergerät 5 kann in einigen Ausführungen vollständig oder teilweise ebenfalls in dem implantierten Pumpengehäuse 4 aufgenommen sein. In anderen Ausführungen ist das Steuergerät 5 extrakorporal angeordnet. Das Pumpengehäuse 4 umfasst außerdem einen mit einer Einlasskanüle des Pumpengehäuses 4 verbundenen Einlasskanal 6, über den Blut aus einer Kammer des Herzens 3 entnommen und über eine Kanüle 7 in ein Blutgefäß 8 gefördert werden kann. Das Steuergerät 5 ist eingerichtet, den Motor der Blutpumpe 2 zum Fördern des Blutes anzusteuern.

Figur 2 zeigt eine weitere schematische Darstellung der Blutpumpe 2. Wiederkehrende Merkmale sind in dieser Abbildung und in den nachfolgenden Abbildungen mit den gleichen Bezugszeichen versehen. Der Elektromotor der Blutpumpe umfasst einen in dem Pumpengehäuse 4 aufgenommenen Stator mit Wicklungen 6. Der Rotor 7 des Elektromotors ist als Förderelement ausgeführt und weist eine Beschaufelung 8, 8' auf. Durch eine in dem dargestellten Bereich im Wesentlichen zylindrische Innenwandung 9 des Pumpengehäuses 4 ist ein Strömungskanal 10 begrenzt, durch den beim Betrieb der Blutpumpe 2 Blut in Richtung der in Fig. 1 gezeigten Kanüle 7 gefördert wird. Um eine Rotation des Rotors 7 zum Fördern des Bluts zu erreichen, wird ein von dem Steuergerät 5 elektronisch kontrollierter Stromfluss in den Wicklungen 6 des Stators erzeugt. Durch das durch den Stromfluss in den Wicklungen 6 erzeugte Magnetfeld kann ein Antriebsmagnet innerhalb des Rotors 7 und damit der gesamte Rotor 7 zur Förderung des Blutes in Drehung versetzt werden. Der Rotor 7 ist durch ein in der Fig. 2 lediglich schematisch dargestelltes Lagerteil 11, das beispielsweise als Gleit-, Kalotten- oder aktives Magnetlager ausgestaltet sein kann, axial und/oder radial gelagert. Zusätzlich ist in dem Pumpengehäuse 4 eine elektronische Steuereinheit 12, beispielsweise zum Auslesen von Sensoren oder zur Steuerung der Blutpumpe, aufgenommen.

Beim Betrieb der Blutpumpe wird durch die Wicklungen 6 des Stators, durch das Lagerteil 11 sowie durch die Steuereinheit 12 Wärme erzeugt, die an einen Blutstrom im Strömungskanal 10 abgegeben wird. In dem Pumpengehäuse 4 ist ein Wärmeverteiler 13 aufgenommen. Zudem ist innerhalb des Rotors 7 ein weiterer Wärmeverteiler 13' aufgenommen. Die von den Wicklungen 6 des Stators, dem Lagerteil 11 sowie der Steuereinheit 12 erzeugte Wärme wird über die Wärmeverteiler 13, 13' auf das Blut im Strömungskanal 10 übertragen. Hierbei wird durch die Wärmeverteiler 13, 13' eine größere Übergangsfläche gewährleistet. Der Wärmeverteiler 13 überträgt hierbei die Wärme über die Innenwandung 9 des Pumpengehäuses 4 auf das Blut. Der weitere Wärmeverteiler 13' überträgt die Wärme über eine Außenwandung 14 des Rotors 7 auf das Blut. Die Außenwandung 14 des Rotors 7 und die Innenwandung 9 des Pumpengehäuses 4, die jeweils den Strömungskanal 10 begrenzen, sind aus Titan oder einer Titanlegierung gefertigt. Die Wärmeverteiler 13, 13' sind beispielsweise aus Kupfer und/oder Aluminium gefertigt und weisen daher eine größere Wärmeleitfähigkeit auf als die Außenwandung 14 und die Innenwandung 9, sodass die Wärmeverteiler 13, 13' eine effiziente Verteilung der Wärme auf eine vergrößerte Fläche der Außenwandung 14 bzw. der Innenwandung 9 bewirken. Eine Wärmeleitfähigkeit der Wärmeverteiler 13, 13' kann beispielsweise 200 oder 400 W/(m K) betragen.

Damit von den oben genannten Wärmequellen abgegebene Wärme nicht an einen Bereich einer Außenseite 21 des Pumpengehäuses 4 abgegeben wird, in dem die Wärme nur unzureichend von Gewebe abgeführt werden kann, das das Pumpengehäuse 4 umgibt, ist ein thermischer Isolator 22 zwischen den Wärmequellen und der Außenseite 21 des Pumpengehäuses 4 vorgesehen.

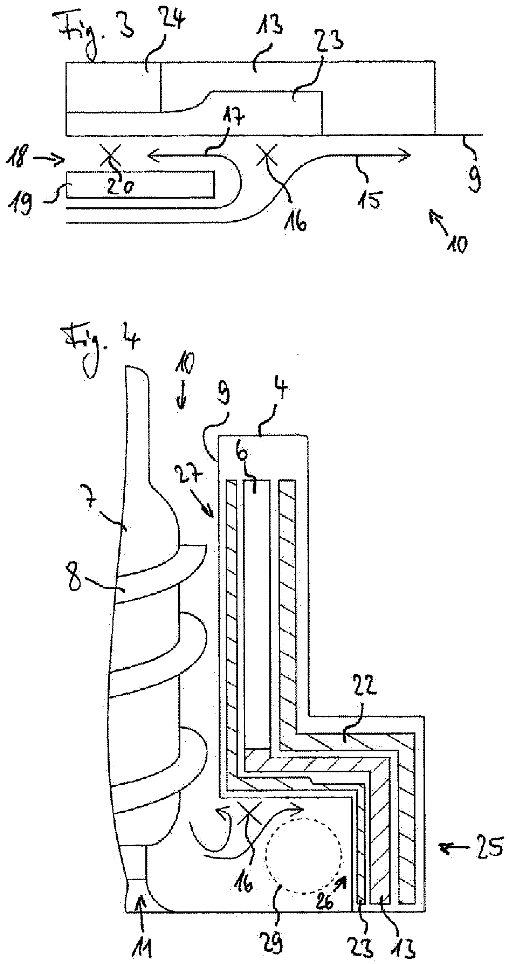

Der Blutstrom in dem Strömungskanal 10 bildet je nach den strukturellen Gegebenheiten der Blutpumpe 2 einen Hauptblutstrom 15 sowie kritische Punkte aus, wie Fig. 3 illustriert. Im Bereich des Hauptblutstroms 15 liegen in der Regel die größten Volumenströme vor. Kritische Punkte im Strömungskanal 10 werden hingegen durch Stagnationspunkte, Rezirkulationspunkte, Verengungspunkte, oder Punkte mechanischer Vorbelastung gebildet. Ein mit Bezugszeichen 16 gekennzeichnetes Kreuz stellt beispielsweise einen Stagnationspunkt dar. An diesem Punkt liegt infolge einer Abspaltung eines Rezirkulationsstromes 17 von dem Hauptblutstrom 15 ein sehr geringer oder nicht vorhandender Volumenstrom vor, so dass eine Aufenthaltsdauer einzelner Blutpartikel in diesem Bereich erhöht ist. Der Rezirkulationsstrom 17 fließt in einem Bereich einer Verengung 18 zwischen der Innenwand 9 des Pumpengehäuses 4 und einem Hindernis 19 in einer dem Hauptblutstrom 15 entgegengesetzten Richtung. In weiteren Ausführungen kann die durch die Innenwand 9 des Pumpengehäuses 4 dargestellte Begrenzung des Strömungskanals 10 durch die Außenwandung 14 des Rotors 7 gebildet werden. Das Hindernis 19 kann in einigen Ausführungen durch einen Teil des Pumpengehäuses 4, ein starr mit dem Pumpengehäuse 4 verbundenes Teil oder durch den Rotor 7, insbesondere durch die Beschaufelung 8, 8' des Rotors 7, gebildet werden. Der Rezirkulationsstrom 17 definiert den Rezirkulationspunkt im Strömungskanal 10, der durch ein Kreuz mit Bezugszeichen 20 gekennzeichnet ist. An dem Rezirkulationspunkt 20 können dieselben Blutpartikel im Betrieb mehrfach vorbeiströmen. Der Rezirkulationspunkt 20 bildet in dem in Fig. 3 dargestellten Beispiel gleichzeitig den Verengungspunkt 20. Der Verengungspunkt 20 kann beispielsweise durch eine Spaltbreite zwischen dem Hindernis 19 und der Innenwandung 9 beziehungsweise Außenwandung 14 von weniger als 2 mm, insbesondere weniger als 1 mm, definiert sein. In Ausführungen, in denen das Hindernis 19 durch die Beschaufelung 8, 8' des Rotors 7 gebildet wird, die im Betrieb rotiert, stellt der Punkt mit Bezugszeichen 20 zugleich einen Punkt mechanischer Vorbelastung des Blutes dar.

Der Wärmeverteiler 13 ist eingerichtet, die auf das Blut im Strömungskanal 10 zu übertragende Wärme von den oben genannten kritischen Punkten 16, 20 fernzuhalten. Es kann zum Beispiel vorgesehen sein, dass der Wärmeverteiler 13 in Bereichen des Hauptblutstroms 15 direkt an einer vom Strömungskanal 10 abgewandten Seite der Wandung 9 anliegt, hingegen in den Bereichen der kritischen Punkte 16, 20 nicht direkt an der Wandung 9 anliegt. In den Bereichen der kritischen Punkte 16, 20 ist ein thermischer Isolator 23 zwischen dem Wärmeverteiler 13 und der Wandung 9 angeordnet, sodass ein Wärmeübertrag auf das Blut in den Bereichen der kritischen Punkte 16, 20 verringert ist. Die verschiedenen thermischen Isolatoren 22, 23 können beispielsweise durch Polyimid-Folien gebildet sein. Es kann in weiteren Ausführungen vorgesehen sein, dass der Wärmeverteiler 13 sich derart erstreckt, dass dieser nicht in den Bereichen der kritischen Punkte 16, 20, sondern lediglich in Bereichen des Hauptblutstroms 15 hinter der Wandung 9 angeordnet und/oder mit dieser verbunden ist.

In der Figur 3 ist die Wärmequelle 24 lediglich schematisch dargestellt. Hierbei kann es sich um jede der verschiedenen oben genannten Wärmequellen handeln. Der Wärmeverteiler 13 ist thermisch leitfähig mit der Wärmequelle 24 verbunden und kann beispielsweise direkt an dieser anliegen oder in einem Abstand von weniger als 1 mm von der Wärmequelle 24 entfernt sein. Es kann auch vorgesehen sein, dass der Wärmeverteiler 13 zur Herstellung eines guten Wärmekontaktes über ein Wärmeleitpaste, einen Wärmeleitkleber oder eine Keramik mit der Wärmequelle 24 verbunden ist.

Figur 4 zeigt eine weitere schematische Ansicht der Blutpumpe 2. Die Blutpumpe weist die durch einen verbreiterten Bereich gebildete Volute 25 auf, die sich durch eine spiral- bzw. schneckenförmige Ausgestaltung beziehungsweise spiral- bzw. schneckenförmige Strömungseigenschaften auszeichnen kann. Im Bereich der Volute 25 ist ein Auslass für das geförderte Blut angeordnet. Eine Position des Auslasses ist schematisch durch einen gestrichelten Kreis mit Bezugszeichen 29 gekennzeichnet. Die Wärmequelle wird in der gezeigten Darstellung durch die in dem Pumpengehäuse 4 aufgenommenen Wicklungen 6 des Stators gebildet. Die Wicklungen 6 sind zwischen den thermischen Isolatoren 22, 23 angeordnet. Die Wicklungen 6 sind über eine thermisch leitfähige und elektrisch isolierende Wärmebrücke, beispielsweise über eine Keramik, mit dem Wärmeverteiler 13 verbunden, sodass im Bereich der Wicklungen 6 durch ohmsche oder magnetische Verluste erzeugte Wärme über den Wärmeverteiler 13 in den Bereich der Volute 25 geleitet wird. Der thermische Isolator 23 weist in dem Bereich der Volute 25 einen dünnen Abschnitt 26, stromaufwärts im Bereich der Wärmequelle sowie im Bereich eines Stagnationspunktes 16 hingegen einen dicken Abschnitt 27 auf, sodass die Wärme zum Großteil im Bereich der Volute 25 auf das Blut im Strömungskanal 10 übertragen wird. Die thermischen Isolatoren 22, 23 sind zudem elektrisch isolierend, um eine hinreichende elektrische Isolation zum Blut bzw. zum Gewebe des Patienten zu gewährleisten.

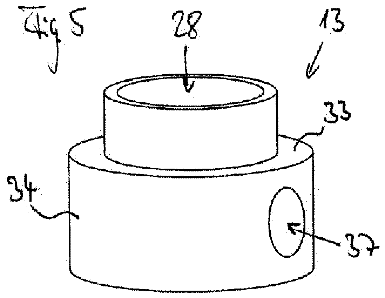

Figur 5 zeigt eine perspektivische Ansicht des bereits oben beschriebenen Wärmeverteilers 13. Der Wärmeverteiler 13 ist zwar im Pumpengehäuse 4 aufgenommen, jedoch ist dieses in Fig. 5 zur besseren Übersicht nicht dargestellt. Der Wärmeverteiler 13 ist flächig, einstückig und ringförmig, sodass der Wärmeverteiler 13 eine durchgehende Öffnung 28 aufweist. In anderen Ausführungen ist der Wärmeverteiler 13 jedoch ringsegmentförmig. Innerhalb der Öffnung 28 sind die nicht dargestellte Innenwandung 9 des Pumpengehäuses 4, der Strömungskanal 10 und der Rotor 7 aufgenommen. Im Bereich der Volute 25 weist der Wärmeverteiler 13 einen Volutenauskleidungsabschnitt 34 mit einem Durchmesser auf, der größer ist als in einem Bereich, der im Bereich der Wärmequelle angeordnet ist. Zwischen diesen Bereichen unterschiedlicher Durchmesser ist ein sich radial erstreckender Abschnitt 33 angeordnet. Somit wird die Wärme durch den Wärmeverteiler 13 auf eine vergrößerte Abgabefläche verteilt. Zudem weist der Wärmeverteiler 13 im Bereich des Auslasses 29 eine sich in radialer Richtung erstreckende Öffnung 37 auf. Eine Dicke des Wärmeverteilers 13 in radialer Richtung kann im Volutenauskleidungsabschnitt 34 beispielsweise 0,5 mm betragen. In axialer Richtung erstreckt sich der Wärmeverteiler 13 beispielsweise über zumindest ein Zehntel, vorzugsweise über zumindest ein Sechstel, einer axialen Ausdehnung des Pumpengehäuses 4. Eine innere Fläche des Wärmeverteilers 13 beträgt beispielsweise 250 mm².

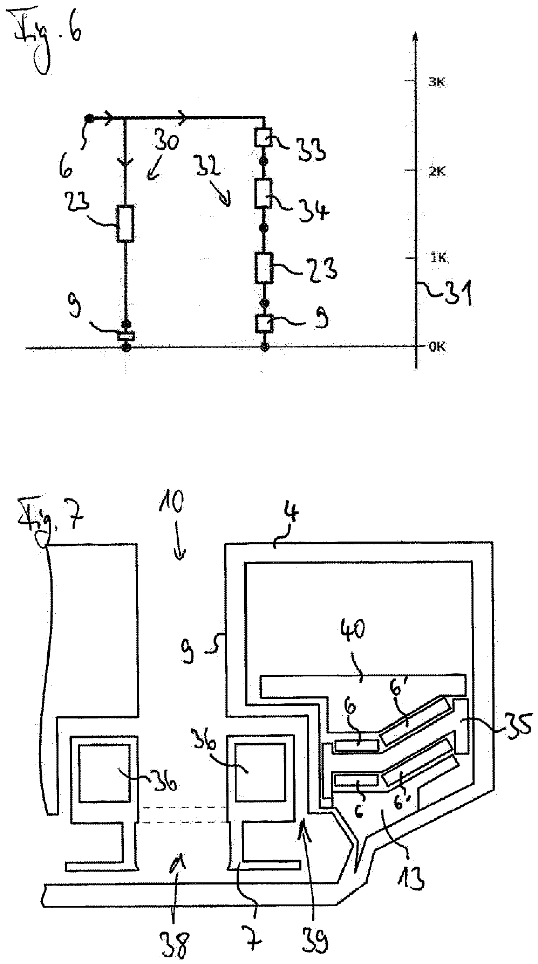

In Fig. 6 ist der Wärmefluss in der Blutpumpe 2 schematisch gezeigt. Im Bereich der Wicklungen 6 des Stators wird beispielsweise eine Wärmeleistung von 3 W erzeugt. Diese Wärmeleistung wird einerseits über einen direkten Pfad 30, d.h. von den Wicklungen 6 radial nach innen über den thermischen Isolator 23 und die Innenwandung 9 des Pumpengehäuses 4 auf das Blut im Strömungskanal 10 übertragen. Auf diesem direkten Pfad wird beispielsweise ein Anteil der gesamten Wärmeleistung von weniger als 50% übertragen. Die vertikale Achse 31 gibt hierbei eine Temperatur oberhalb einer Temperatur des Blutes an. Zudem wird die Wärmeleistung auf einem zweiten Pfad 32 auch über den Wärmeverteiler 13 und hier insbesondere seinen radialen Abschnitt 33 und seinen Volutenauskleidungsabschnitt 34, den thermischen Isolator 23 im Bereich der Volute 25 sowie die Innenwandung 9 des Pumpengehäuses 2 im Bereich der Volute 25. Auf dem zweiten Pfad 32 kann eine größere Wärmeleistung abgeführt werden als auf dem direkten Pfad 30. Beispielsweise kann eine über den zweiten Pfad 32 an das Blut abgegebene Wärmeleistung 1,7 W betragen.

Eine weitere Ausgestaltung einer Blutpumpe 2 ist in Fig. 7 dargestellt. Der Rotor 7 dieser Blutpumpe 2 weist eine in axialer Richtung verlaufende Öffnung 38 auf, durch die ein nach unten gerichteter Hauptblutstrom gefördert wird. In einem Spalt 39 zwischen dem Rotor 7 und der Innenwandung 9 des Pumpengehäuses 4 tritt ein Rezirkulationsstrom auf, der in einer Richtung, die dem Hauptblutstrom entgegengerichtet ist, fließt. Der Rotor 7 weist in seinem Inneren einen Rotormagneten 36 auf, über den eine Drehung des Rotors 7 zur Förderung von Blut durch den Stator angetrieben werden kann. Der Stator ist im Pumpengehäuse 4 aufgenommen und umfasst Wicklungen 6, 6' und einen Magnetkern 35, beispielsweise Eisen. Zudem ist in dem Pumpengehäuse 4 eine Elektronik 40 aufgenommen. Im Betrieb kann sowohl durch ohmsche Verluste in der Elektronik 40 und in den Wicklungen 6, 6' als auch durch Ummagnetisierungsverluste in dem Magnetkern 35 Wärme erzeugt wird, sodass die Elektronik 40, die Wicklungen 6, 6' und der Magnetkern 35 Wärmequellen darstellen.

Zur effizienten und sicheren Abgabe der Wärme von den Wärmequellen auf das Blut weist die Blutpumpe 2 den Wärmeverteiler 13 auf. Der Wärmeverteiler 13 verteilt die Wärme von den Wärmequellen auf eine vergrößerte Fläche der Innenwandung 9 des Pumpengehäuses 4 im Bereich der Volute 25. Der Wärmeverteiler 13 ist wie oben genauer erläutert ausgeführt und kann die thermische Blutbelastung im Spalt und Rezirkulationsgebiet 39 verringern.

Lediglich in den Ausführungsbeispielen offenbarte Merkmale der verschiedenen Ausführungsformen können miteinander kombiniert und einzeln beansprucht werden.

## Patentansprüche

1. Implantierbare Blutpumpe (2) zum Unterstützen einer Herzfunktion, umfassend eine Wärmequelle (24) und eine Wandung (9, 14), die einen Strömungskanal (10) begrenzt,
**gekennzeichnet durch** einen Wärmeverteiler (13) zum Verteilen von durch die Wärmequelle (24) erzeugter Wärme auf eine Fläche der Wandung (9, 14), wobei der Wärmeverteiler (13) zur Übertragung von Wärme von der Wärmequelle (24) zu in dem Strömungskanal (10) gefördertem Blut thermisch leitfähig mit der Wärmequelle (24) und thermisch leitfähig mit einer von dem Strömungskanal (10) abgewandten Seite der Wandung (9, 14) verbunden ist.

2. Implantierbare Blutpumpe (2) nach Anspruch 1, **dadurch gekennzeichnet, dass** der Wärmeverteiler (13) auf einer Seite, die der von dem Strömungskanal (10) abgewandten Seite der Wandung (9, 14) zugewandt ist, eine Fläche von mindestens 50 mm² aufweist.

3. Implantierbare Blutpumpe (2) nach einem der Ansprüche 1 oder 2,
**dadurch gekennzeichnet, dass** der Wärmeverteiler (13) eine Wärmeleitfähigkeit aufweist, die größer ist als eine Wärmeleitfähigkeit der Wandung (9, 14).

4. Implantierbare Blutpumpe (2) nach einem der Ansprüche 1 bis 3, **dadurch gekennzeichnet, dass** der Wärmeverteiler (13) ein Material mit einer Wärmeleitfähigkeit von zumindest 50 W/(m K) oder zumindest 100 W/(m K) enthält.

5. Implantierbare Blutpumpe (2) nach einem der Ansprüche 1 bis 4, **dadurch gekennzeichnet, dass** der Wärmeverteiler (13) so angeordnet ist, dass dieser eingerichtet ist, die Wärme der Wärmequelle (24) zu einem Bereich eines Hauptblutstroms (15) im Strömungskanal (10) zu übertragen.

6. Implantierbare Blutpumpe (2) nach einem der Ansprüche 1 bis 5, **dadurch gekennzeichnet, dass** der Wärmeverteiler (13) so angeordnet ist, dass dieser eingerichtet ist, die Wärme von zumindest einem Stagnationspunkt (16) und/oder zumindest einem Rezirkulationspunkt (20) fernzuhalten.

7. Implantierbare Blutpumpe (2) nach einem der Ansprüche 1 bis 6, **gekennzeichnet durch** einen thermischen Isolator, der in einem Bereich zumindest eines Stagnationspunktes (16) und/oder zumindest eines Rezirkulationspunktes (20) zwischen der von dem Strömungskanal (10) abgewandten Seite der Wandung (9, 14) und dem Wärmeverteiler (13) angeordnet ist.

8. Implantierbare Blutpumpe (2) nach einem der Ansprüche 1 bis 7, **dadurch gekennzeichnet, dass** der Wärmeverteiler (13) über eine Wärmebrücke, die insbesondere eine Wärmeleitpaste, einen Wärmeleitkleber, ein Metall oder eine Keramik umfasst, mit der Wärmequelle (24) verbunden ist.

9. Implantierbare Blutpumpe (2) nach einem der Ansprüche 1 bis 8, **gekennzeichnet durch** ein implantierbares Pumpengehäuse (4), wobei die Wandung eine Innenwandung (9) des Pumpengehäuses (4) ist.

10. Implantierbare Blutpumpe (2) nach Anspruch 9, **dadurch gekennzeichnet, dass** der Wärmeverteiler (13) die Innenwandung (9) des Pumpengehäuses (4) über einen Winkelbereich von zumindest 30 Grad, insbesondere zumindest 90 Grad umgibt.

11. Implantierbare Blutpumpe (2) nach einem der Ansprüche 1 bis 10, **gekennzeichnet durch** einen Motor, wobei die Wärmequelle (24) ein Motorteil des Motors ist.

12. Implantierbare Blutpumpe (2) nach Anspruch 11, **dadurch gekennzeichnet, dass** der Motorteil ein Stator des Motors ist.

13. Implantierbare Blutpumpe (2) nach einem der Ansprüche 1 bis 12, **gekennzeichnet durch** einen Rotor (7), wobei die Wärmequelle (24) ein Lagerteil (11) zur Lagerung des Rotors (7) ist.

14. Implantierbare Blutpumpe (2) nach Anspruch 13, wobei die Wandung eine Außenwandung (14) des Rotors (7) ist.

15. Implantierbare Blutpumpe (2) nach einem der Ansprüche 9 bis 14, **dadurch gekennzeichnet, dass** das Pumpengehäuse (4) eine Volute (25) umfasst, wobei die Innenwandung (9) eine Innenwandung der Volute (25) ist.
